(19) Europäisches Patentamt
European Patent Office
Office européen des brevets

(11) **EP 3 457 820 B1**

(12) **EUROPEAN PATENT SPECIFICATION**

(45) Date of publication and mention of the grant of the patent:
**14.10.2020 Bulletin 2020/42**

(21) Application number: **16901693.8**

(22) Date of filing: **13.05.2016**

(51) Int Cl.:
***H05H 1/30*** *(2006.01)* ***A61B 18/18*** *(2006.01)*
***A61N 5/00*** *(2006.01)* ***A61N 5/06*** *(2006.01)*
***A61N 5/10*** *(2006.01)* ***A61B 90/30*** *(2016.01)*
***A61B 18/04*** *(2006.01)* ***A61N 1/44*** *(2006.01)*
***H05H 1/24*** *(2006.01)*

(86) International application number:
**PCT/JP2016/064260**

(87) International publication number:
**WO 2017/195345 (16.11.2017 Gazette 2017/46)**

(54) **MEDICAL PLASMA-GENERATING DEVICE**

VORRICHTUNG ZUR ERZEUGUNG VON MEDIZINISCHEM PLASMA

DISPOSITIF DE GÉNÉRATION DE PLASMA MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.03.2019 Bulletin 2019/12**

(73) Proprietor: **Fuji Corporation**
**Chiryu-shi, Aichi 472-8686 (JP)**

(72) Inventors:
- **JINDO, Takahiro**
  **Chiryu,**
  **Aichi, (JP)**
- **TAKIKAWA, Shinji**
  **Chiryu**
  **Aichi (JP)**

(74) Representative: **Grünecker Patent- und Rechtsanwälte**
**PartG mbB**
**Leopoldstraße 4**
**80802 München (DE)**

(56) References cited:
**WO-A1-2010/082561**
**WO-A1-2014/184910**
**WO-A1-2014/184910**
**JP-A- H08 745**
**JP-A- 2002 540 861**
**JP-A- 2009 018 260**
**JP-A- 2010 129 198**
**JP-A- 2010 129 198**
**JP-A- 2013 252 540**
**US-A1- 2011 298 376**
**US-A1- 2014 188 195**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a medical-use plasma generating device for use in plasma medical treatments.

### Background Art

Plasma processing is performed by a plasma generating device ejecting plasma from an emission port towards a processing target such that plasma is applied to the processing target. Examples of a plasma generating device is disclosed in the patent literature below.

Patent literature 1: WO2013/0108797

Patent literature 2: JP-A-2012-059548

The Japanese patent application JP 2010 129198 A discloses a plasma generating device comprising: a plasma ejecting device configured to eject plasma; and an inert gas ejecting device configured to eject an inert gas such that an outer circumference of the plasma ejected by the plasma ejecting device is covered by the inert gas, wherein is the device is provided with a tubular outer housing and a tubular inner housing with an outer dimension smaller than an inner dimension of the outer housing, the inner housing being inserted inside the outer housing.

### Summary of Invention

### Technical Problem

Plasma includes active radicals and research is progressing of emitting plasma for medical treatment purposes, wherein the surface of a processing body is activated by plasma being applied. Specifically, for example, oxygen plasma generated by using oxygen as a processing gas is effective for killing cells, and by applying oxygen plasma to cancerous cells, emitting of such plasma can be used for the purpose of cancer treatment. Also, for example, hydrogen plasma generated by using hydrogen as a processing gas is effective for regenerating cells, and by applying hydrogen plasma to skin, emitting of such plasma can be used for the purpose of skin regeneration treatment. In such ways, emitting of plasma is performed for medical treatment purposes, but for medical treatment, the concentration of the processing gas and so on is controlled strictly. However, because active radicals in plasma are unstable, there is a tendency for the effectiveness of the plasma to be lowered when the plasma is being applied to the processing target Also, because plasma is invisible, it is difficult to apply plasma to the processing target appropriately. Thus, there remains considerable room for improvement of a medical-use plasma generating device, and the practicality of a medical-use plasma generating device may be improved by applying various refinements. The present invention takes account of such problems, and an object thereof is to provide a medical-use plasma generating device with high practicality.

### Solution to Problem

To solve the above problems, a medical-use plasma generating device of the present invention is defined in the appended claims. The following disclosure contains illustrative embodiments for a better understanding of the invention. The methods disclosed do not form part of the claimed invention. The disclosure includes: a plasma ejecting device configured to eject plasma; and an inert gas ejecting device configured to eject an inert gas such that an outer circumference of the plasma ejected by the plasma ejecting device is covered by the inert gas.

### Advantageous Effects

With an atmospheric pressure plasma generating device of the present disclosure, inert gas is ejected to surround an outer circumference of ejected plasma. Thus, the ejected plasma is shielded by the inert gas such that the plasma can be applied to a processing target, that is, an affected body part, without being exposed to air. Accordingly, deactivation of the plasma by air is prevented, and plasma medical treatment can be performed without decreasing the effect that is expected based on a strictly managed concentration of the processing gas.

Also, with a plasma emitting method of the present disclosure, which does not fall within the scope of the claims, plasma is ejected after the inert gas is ejected. Thus, before plasma is ejected, inert gas is ejected to cover the plasma emission port, meaning that the plasma is ejected through the inert gas. This means it is possible to reliably block air from contacting plasma ejected from the emission port.

### Brief Description of Drawings

Fig. 1

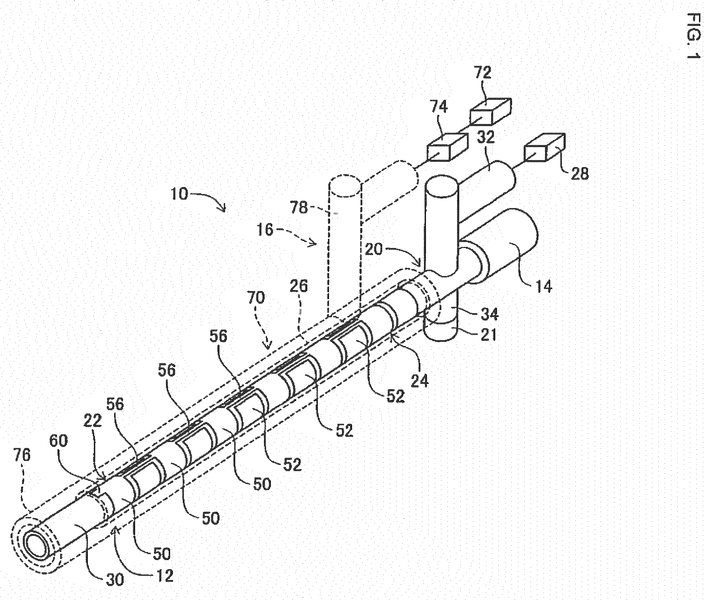

Fig. 1 is a perspective view of a plasma emitting device.
Fig. 2

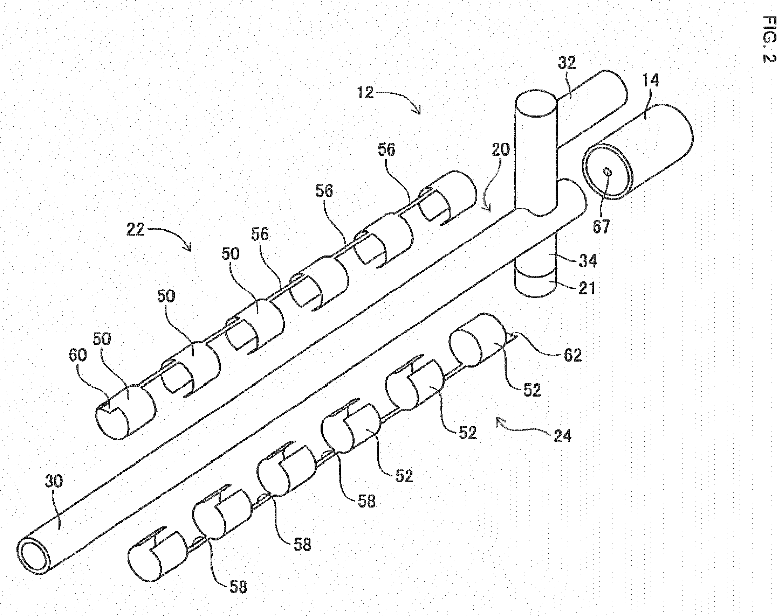

Fig. 2 is an exploded view of a plasma ejecting device.
Fig. 3

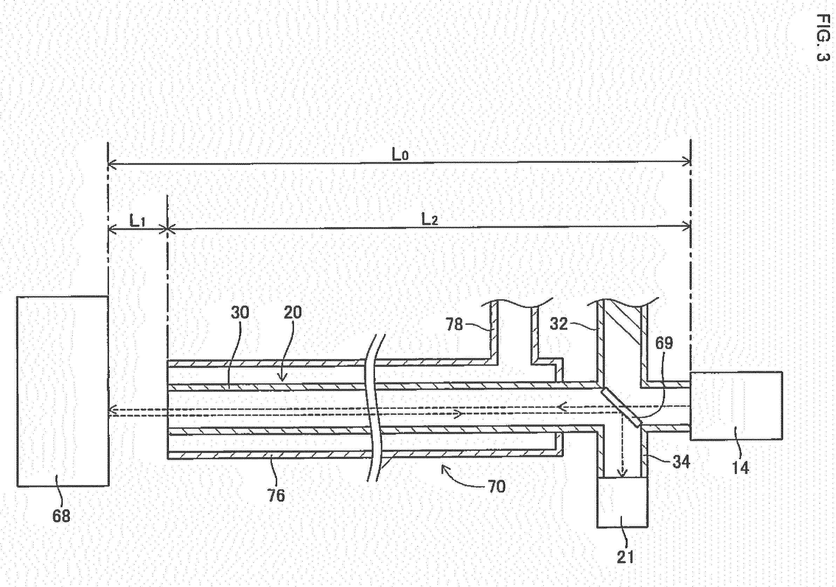

Fig. 3 is a cross section view of the plasma emitting device.
Fig. 4

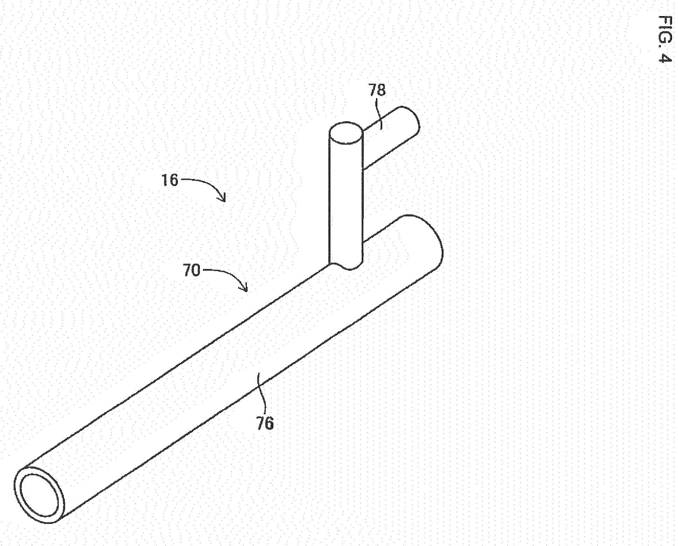

Fig. 4 is a perspective view of an inert gas ejecting device.
Fig. 5

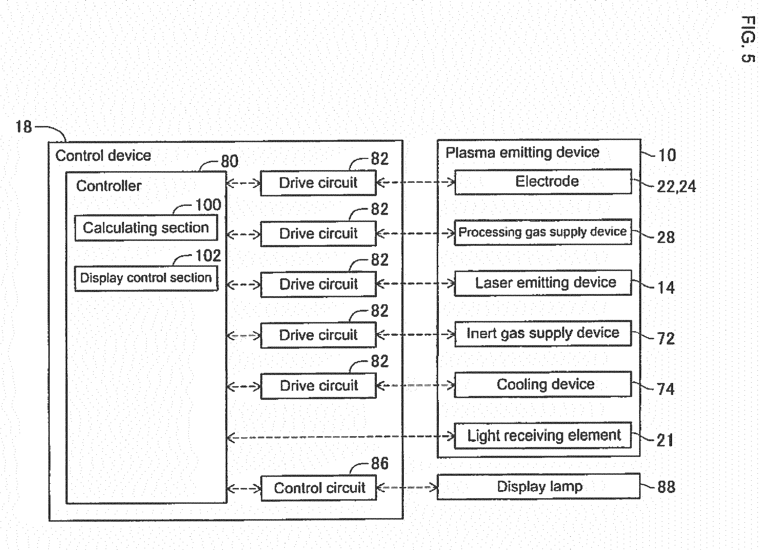

Fig. 5 is a block diagram showing a control device.

Fig. 6

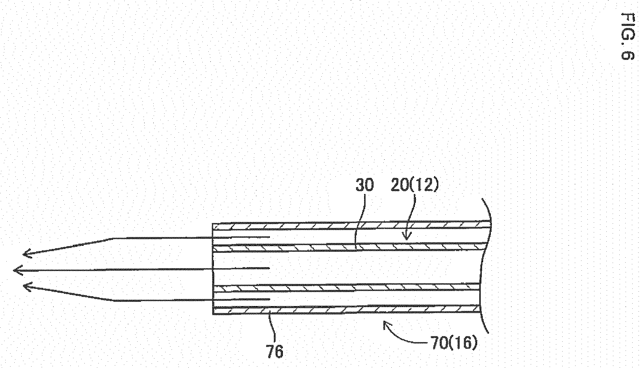

Fig. 6 illustrates the flow of plasma and inert gas.

Description of Preferred Embodiments

The following describes in detail referring to the figures an example embodiment of the present invention.

Configuration of plasma emitting device

Fig. 1 shows an embodiment of the present invention, plasma emitting device 10. Plasma emitting device 10 is for medical use and is for applying plasma to an affected body part. Plasma emitting device 10 is provided with plasma ejecting device 12, laser emitting device 14, inert gas ejecting device 16, and control device 18 (refer to fig. 5).

Plasma ejecting device 12 is for ejecting plasma and is provided with main body 20, light receiving element 21, pair of electrodes 22 and 24, glass tube 26, and processing gas supply device 28. Main body section 20 is formed from sapphire glass and is configured from cylindrical section 30, bent section 32, and dividing section 34. Cylindrical section 30 is substantially a round tube. Bent section 32 is a tube bent into an L-shape, and an end section thereof is connected in an upright state to an outer surface of cylindrical section 30 near an end of cylindrical section 30. The inside of cylindrical section 30 and the inside of bent section 32 are linked. Dividing section 34 is a short cylinder and is connected in an upright state to cylindrical section 30 on the opposite side to bent section 32. The inside of cylindrical section 30 and the inside of dividing section 34 are linked. Light receiving element 21 is arranged on the lower end of dividing section 34. Light receiving element 21, to be described in detail later, functions as a light receiving sensor, and a distance between a processing target and plasma emitting device 10 is calculated based on the received light.

Multiple electrical discharge sections 50 and 52 of a pair of electrodes 22 and 24 are vacuum deposited on the outer circumferential surface of cylindrical section 30 of main body 20 so as to be lined up alternately in an axis direction of cylindrical section 30. In detail, as shown in fig. 2, electrode 22 includes multiple electrical discharge sections 50 and multiple connecting sections 56, and electrode 24 includes multiple electrical discharge sections 52 and multiple connecting sections 58. Note that, fig. 2 is a theoretical view showing electrodes 22 and 24 removed from cylindrical section 30.

The multiple electrical discharge sections 50 of electrode 22 are vacuum deposited on the outer circumferential surface of cylindrical section 30 extending in the circumferential direction, and are arranged at a specified interval lined up in the axis direction of cylindrical section 30. Also, connecting sections 56 of electrode 22 are vacuum deposited on the outer circumferential surface of cylindrical section 30 extending in a line in the axis direction of cylindrical section 30, and are connected to the multiple electrical discharge sections 50. Note that, from among the multiple electrical discharge sections 50 of electrode 22, electrical discharge section 50 positioned at one end is vacuum deposited around the entire circumference in the circumferential direction of cylindrical section 30; the other electrical discharge sections 50 are vacuum deposited extending in the circumferential direction of cylindrical section 30, except for a portion on the opposite side to connecting section 56. Current passing section 60 is formed on the electrical discharge section 50 vacuum deposited across the entire circumference in the circumferential direction of cylindrical section 30 protruding from an end of cylindrical section 30.

Further, the multiple electrical discharge sections 52 of electrode 24 are vacuum deposited on the outer circumferential surface of cylindrical section 30 extending in the circumferential direction, and are arranged lined up in the axis direction of cylindrical section 30 so as to be positioned between the multiple electrical discharge sections 50 of electrode 22. Note that, from among the multiple electrical discharge sections 52 of electrode 24, electrical discharge sections 52 positioned between two of the electrical discharge sections 50 of electrode 22 are vacuum deposited extending in the circumferential direction of cylindrical section 30 excluding connecting section 56 of electrode 22; the remaining electrical discharge sections 52 positioned at the ends are vacuum deposited across the entire circumference in the circumferential direction of cylindrical section 30. Current passing section 62 is formed on the electrical discharge section 52 vacuum deposited across the entire circumference in the circumferential direction of cylindrical section 30 protruding from an end of cylindrical section 30. Also, connecting sections 58 of electrode 24 are vacuum deposited on the outer circumferential surface of cylindrical section 30 extending in a line in the axis direction of cylindrical section 30 at locations where electrical discharge sections 50 of electrode 22 are not vacuum deposited, and are connected to the multiple electrical discharge sections 52. Thus, the pair of electrodes 22 and 24 have electrical discharge sections 50 of electrode 22 and electrical discharge sections 52 of electrode 24 vacuum deposited on the outer circumferential surface of cylindrical section 30 lined up alternately with a specified gap between them.

As shown in fig. 1, glass tube 26 is arranged on the outer circumferential surface of cylindrical section 30 of main body 20 so as to entirely cover the pair of electrodes 22 and 24 vacuum deposited on the outer circumferential surface of cylindrical section 30. By this, it is possible to prevent exposure of electrodes 22 and 24, through which high voltage is applied, thereby maintaining safety. Note that, because electrodes 22 and 24 are encased by glass tube 26, glass tube 26 encroaches in between electrical discharge sections 50 of electrode 22 and electrical discharge sections 52 of electrode 24.

Gas supply device 28 supplies processing gas and is connected to an end of bent section 32 opposite to an end of bent section 32 that is connected to cylindrical section 30. Thus, processing gas is supplied inside cylindrical section 30 via bent section 32. Note that, processing gas may be gas in which an inert gas such as nitrogen or argon is mixed with active gases in the air such as oxygen at a given ratio, or may be only an inert gas, or only an active gas. Also, processing gas supply device 28 may also be provided with a function to heat or cool the processing gas, such that processing gas can be supplied at a given temperature.

Laser emitting device 14 emits laser light and is substantially a short cylinder. An end surface of laser emitting device 14 is coaxially connected to an end surface of cylindrical section 30 at which bent section 32 is arranged. In a central portion of the end surface of laser emitting device 14 connected to cylindrical section 30, emitting hole 67 (refer to fig. 2) is formed, and laser emitting device 14 emits laser light from emitting hole 67 in an axial direction of cylindrical section 30.

Note that, laser light is monochromatic and has directionality and coherence. Directionality means that emitted light travels substantially straight without being spread. Being monochromatic means that the wavelength of the light is narrow, that is, of a single color. Coherence is related to a fixed relationship between the amplitude and phase of two or more waves of light, wherein interference fringes occur when the two or more waves of light interfere. Laser light with such qualities converges in a narrow range. Note that, laser light emitted from laser emitting device 14 is red light with a visible wavelength.

By arranging laser emitting device 14 at an end of cylindrical section 30 of plasma emitting device 12 in this manner, as shown in fig. 3, laser light emitted from laser emitting device 14 passes through the inside of cylindrical section 30, and is emitted towards the outside of plasma emitting device 10 from the end of cylindrical section 30 opposite to the end at which laser emitting device 14 is provided (also referred to as the "front end" of cylindrical section 30). Thus, in a case in which processing target 68 is positioned opposite the front end of cylindrical section 30, laser light emitted by laser emitting device 14 is applied to processing target 68.

Reflector 69 is arranged inside cylindrical section 30 opposite to dividing section 34. Reflector 69 is arranged at a 45 degree angle with respect to the axis direction of cylindrical section 30, and the reflective surface faces towards dividing section 34. Note that, the reflectance of reflector 69 is 50%, and the permeability is 50%. Therefore, 50% of the laser light emitted from laser emitting device 14 passes through reflector 69 and is emitted outside of plasma emitting device 10. Therefore, 50% of the laser light emitted from laser emitting device 14 is applied to processing target 68. Light applied to processing target 68 is reflected by processing target 68, and that reflected light is reflected inside cylindrical section 30 coaxially to the laser light emitted from laser light emitting device 14. The reflected light that goes through the inside of cylindrical section 30 is reflected by reflector 69 and goes through the inside of dividing section 34. The reflected light is then detected by light receiving element 21 and the distance between plasma emitting device 10 and processing target 68 is calculated based on the detected light. Details about calculation of the distance between plasma emitting device 10 and processing target 68 are described later.

Inert gas ejecting device 16 is for ejecting an inert gas such as nitrogen, argon, or helium, and is arranged to cover cylindrical section 30 of plasma ejecting device 12. Inert gas ejecting device 16 is provided with main body 70, inert gas supply device 72, and cooling device 74. As shown in fig. 4, main body 70 is configured from cylindrical section 76 and bent section 78. Cylindrical section 76 is substantially a round tube with an inside diameter larger than the outside diameter of cylindrical tube 30 of plasma ejecting device 12. Cylindrical section 30 of plasma ejecting device 12 is inserted inside cylindrical section 76 with a clearance gap between the outside surface of cylindrical section 30 and the inside surface of cylindrical section 76.

Cylindrical section 76 is shorter than cylindrical section 30 of plasma ejecting device 12. Also, as shown in fig. 1, the end surface of the front end of cylindrical section 30 of plasma ejecting device 12 is aligned with the end surface of cylindrical section 76. The other end of cylindrical section 76 is positioned towards bent section 32 and dividing section 34 of plasma ejecting device 12 and is blocked up.

Bent section 78 is a tube bent into an L-shape, and an end section thereof is connected in an upright state to an outer surface of cylindrical section 76 near the end of cylindrical section 76 towards bent section 32 and dividing section 34. The inside of cylindrical section 76 and the inside of bent section 78 are linked.

Inert gas supply device 72 is connected, via cooling device 74, to the end of bent section 78 opposite to the end of bent section 78 connected to cylindrical section 76. Inert gas supply device 72 supplies inert gas, and cooling device 74 cools inert gas supplied by inert gas supply device 72. Thus, cooled inert gas is supplied inside cylindrical section 76 via bent section 78.

As shown in fig. 5, control device 18 is provided with controller 80, multiple drive circuits 82, and control circuit 86. The multiple drive circuits 82 are connected to electrodes 22 and 24, processing gas supply device 28, laser emitting device 14, inert gas supply device 72, and cooling device 74. Controller 80 is provided with a CPU, ROM, RAM, and so on, is formed mainly from a computer, and is connected to the multiple drive circuits 82. By this, operation of plasma emitting device 10 is controlled by controller 80. Controller 80 is also connected to light receiving element 21. Controller 80 acquires information related to light received by light receiving element 21. Controller 80 is also connected to display lamp 88 via control circuit 86. Display lamp 88 is a lamp that displays a red or green light and controller 80 causes display lamp 88 to shine red or green.

Emitting plasma using the plasma emitting device

According to the above configuration, with plasma emitting device 10, plasma is ejected from the front end of cylindrical section 30 of plasma ejecting device 12 to be applied to processing target 68. Plasma includes active radicals and is used in medical fields for the purpose of treating an affected body part with active radicals. Specifically, for example, by using oxygen as a processing gas, oxygen plasma is applied to processing target 68. Oxygen plasma is effective at killing cells, and, for example, by applying the oxygen plasma to cancerous cells, can be used for the purpose of cancer treatment. Also, for example, by using hydrogen as a processing gas, hydrogen plasma can be applied to processing target 68. Hydrogen plasma is effective for regenerating cells, and, for example, by applying the hydrogen plasma to skin, can be used for the purpose of skin regeneration treatment.

Specifically, in plasma emitting device 10, processing gas is supplied by processing gas supply device 28 inside cylindrical section 30 via bent section 32. Because the end of cylindrical section 30 at which bent section 32 is arranged is covered by laser emitting device 14, processing gas supplied to cylindrical section 30 flows from that end towards the opposite end. That is, processing gas flows towards the inside of cylindrical section 30 on which electrodes 22 and 24 are vacuum deposited.

Voltage is applied to current passing sections 60 and 62 of electrodes 22 and 24 such that current flows through electrodes 22 and 24. By this, electrical discharge is generated between electrical discharge sections 50 and 52 of the pair of electrodes 22 and 24. Here, because electrodes 22 and 24 are encased by glass tube 26, which is an insulating body, electrical discharge is generated inside cylindrical section 30 such that the processing gas flowing inside cylindrical section 30 is plasmarized. Thus, plasma is emitted in an axial direction of cylindrical section 30 from an opening (also referred to as a "plasma emission port") of cylindrical section 30 formed in an end surface of cylindrical section 30 opposite to an end of cylindrical section 30 to which laser emitting device 14 is connected. Here, a person giving treatment, such as a doctor, points the plasma emission port towards the affected body part of the patient to treat the affected body part by applying plasma.

Note that, when plasma is applied to the affected body part, the concentration of active radicals in the applied plasma should be appropriate in order to perform appropriate treatment, therefore the concentration of processing gas supplied from processing gas supply device 28 is appropriately controlled. However, if air contacts the plasma from when the plasma is ejected from the plasma emission port of plasma emitting device 10 to when the plasma is applied to the affected body part, active radicals will deactivate, and the concentration of plasma active radicals applied to the affected body part may no longer be appropriate for treatment. In such a case, appropriate treatment cannot be guaranteed. Also, the effectiveness of treatment is lowered by the deactivation of the plasma.

Considering this, with plasma emitting device 10, when applying plasma to an affected body part, inert gas is ejected together with the plasma. In detail, inert gas is supplied from inert gas supply device 72 to bent section 78 via cooling device 74. Here, inert gas supplied from inert gas supply device 72 is cooled by cooling device 74. Thus, cooled inert gas is supplied inside cylindrical section 76 via bent section 78. Because the end of cylindrical section 76 at which bent section 78 is arranged is blocked up, processing gas supplied to cylindrical section 76 flows from that end towards the opposite end. Here, the inert gas flows between the inner surface of cylindrical section 76 and the outer surface of cylindrical section 30. Also, inert gas is ejected from the opening (also referred to as "inert gas emission port") of cylindrical section 76 at the end of cylindrical section 76 opposite to the end at which bent section 78 is arranged.

Because inert gas is ejected between the inner circumferential surface of cylindrical section 76 and the outer circumferential surface of cylindrical section 30, inert gas ejected from the inert gas emission port has a cylindrical shape. Plasma is ejected through the inside of the inert gas ejected in a cylindrical shape. That is, the inert gas is ejected so as to cover the outer circumference of the ejected plasma. Thus, the ejected plasma is shielded by the inert gas such that the plasma can be applied to the affected body part without being exposed to air. Thus, an appropriate concentration of plasma can be applied to the affected body part thereby enabling effective treatment. Also, effective treatment is enabled by preventing deactivation of the plasma.

Because it is desirable to reliably block the plasma from contacting the air, plasma is ejected from the plasma emission port after inert gas is ejected from the the inert gas emission port. That is, first inert gas is supplied from inert gas supply device 72 such that inert gas is ejected from the inert gas emission port. Then, after the inert gas has started to be ejected from the inert gas emission port, processing gas is supplied from processing gas supply device 28 and voltage is applied to the pair of electrodes 22 and 24. Therefore, inert gas is ejected in a cylindrical shape to cover the plasma emission port before plasma starts to be ejected from the plasma emission port, and the plasma is then ejected inside the cylindrical inert gas. Thus, plasma ejected from the plasma emission port is reliably blocked from contacting the air.

Further, the ejection speed of the inert gas ejected from the inert gas emission port is faster than the ejection speed of the plasma ejected from the plasma emission port such that spreading of the inert gas is prevented. In detail, if a gas (referred to as a "first gas") flowing at a given speed and a gas (referred to as a "second gas") flowing a speed faster than the given speed are flowing in the same direction, friction arises between the first gas and the second gas, and the second gas flows towards the first gas. Therefore, by making the ejection speed of the inert gas faster than the ejection speed of the plasma, as shown in fig. 6, inert gas (second gas) ejected from the inert gas emission port (between the inner circumferential surface of main body 70 and the inner circumferential surface of cylindrical section 30) flows towards the plasma (first gas) ejected from the plasma emission port (inside of cylindrical section 30). Thus, the inert gas converges on the plasma, preventing the inert gas from spreading. This prevents the shield around the plasma being broken due to spreading of the inert gas. Note that, the ejection speed of the plasma and the ejection speed of the inert gas are adjusted by adjusting the amount of gas supplied by processing gas supply device 28 and inert gas supply device 72.

Also, plasma ejected from the plasma ejection port has a tendency to become hot due to the high voltage electrical discharge, but the inert gas shielding the ejected plasma is cooled gas. Therefore, the temperature of the plasma ejected from the plasma emission port is lowered, preventing hot plasma being applied to the affected body part.

Note that, because the wavelength of the plasma is in a vacuum ultraviolet range, it cannot be checked visually. Therefore, the plasma may not be applied to the affected body part appropriately. Considering this, plasma emitting device 10 is provided with laser emitting device 14, and by the laser light emitted by laser emitting device 14, the application position of the plasma can be checked.

In detail, as described above, laser emitting device 14 is connected to an end surface of cylindrical section 30 of plasma ejecting device 12, and emits laser light in an axial direction of cylindrical section 30. That is, laser emitting device 14 emits laser light coaxially with the emission direction of plasma. Also, laser light is directional light that travels straight. Thus, the laser light emitted from laser emitting device 14 passes through cylindrical section 30 and is emitted from the plasma emission port of cylindrical section 30 coaxially to the ejection direction of the plasma. By this, as shown in fig. 3, laser light is applied to a location to which the plasma is applied. Because the laser light is red laser light of a high frequency in a visible range, the person providing treatment is able to appropriately apply plasma to the processing target 68 while visually checking the laser light.

However, if the processing target 68 to which plasma is to be applied is too far away from plasma emitting device 10, the plasma may not reach the processing target 68, thereby lowering the effectiveness of the plasma treatment. On the other hand, it is undesirable for the processing target 68 to be too close to plasma emitting device 10 if the plasma is hot. Thus, it is desirable to make the distance between plasma emitting device 10 and processing target 68 appropriate (also referred to as the "processing target separation distance") when applying plasma to the processing target 68. Therefore, with plasma emitting device 10, the processing target separation distance is calculated using the laser light that is used for checking the application position of the plasma.

In detail, plasma emitting device 10 is provided with light receiving element 21 and, as shown in fig. 3, laser light emitted towards processing target 68 from laser emitting device 14 is reflected and the reflected light is received by light receiving element 21. AM (amplitude modulation) is performed on the laser light emitted from laser emitting device, and the amplitude of amplitude modulated laser light changes over time. Therefore, a phase difference arises between a wave of laser light emitted from laser emitting device 14 and a wave of laser light received by laser receiving element 21, with that phase difference being calculated by controller 80. The phase difference is proportional to the travel distance of the light received by light receiving element 21 that was emitted from laser emitting device 14 towards processing target 68 and reflected by processing target 68, that is, is proportional to the emission distance of the laser light (also referred to as "laser emission distance").

Note that, the distance between laser emitting device 14 and reflector 69 is the same as the distance between reflector 69 and light receiving element 21. Therefore, the laser emission distance is $2 \times L_0$, and controller 80 calculates the laser emission distance ($2 \times L_0$) based on the phase difference. Length $L_2$ of cylindrical section 30 is memorized in controller 80. Therefore, by subtracting the length of cylindrical section 30 ($L_2$) from half of the laser emission distance ($2 \times L_0$), distance $L_1$ between the plasma emission port of cylindrical section 30 and processing target 68, that is, the processing target separation distance, can be calculated.

In controller 80, after the processing target separation distance is calculated, it is determined whether the processing target separation distance is within a predetermined set range. If the calculated processing target separation distance is within the set range, controller 80 causes display lamp 88 to display a green light; if the calculated processing target separation distance is not within the set range, controller 80 causes display lamp 88 to display a red light. The set range can be entered by an operator via an input device (not shown) and can be set to any range. Therefore, by entering an appropriate range for plasma treatment as the set range, an operator can guarantee appropriate plasma treatment. That is, a person giving treatment can move plasma emitting device 10 towards the affected body part, that is, processing target 68, while applying plasma to the processing target 68 such that a green light is always displayed by display lamp 88, thereby guaranteeing appropriate plasma treatment.

In this manner, with plasma emitting device 10, it is possible to check the plasma application position using the laser light. Also, the processing target separation distance can be calculated using the laser light used for checking the plasma application position, and whether the processing target separation distance is appropriate can be reported to the person performing treatment Thus, the person performing treatment can perform treatment while checking the plasma application position and the plasma emission distance, thereby guaranteeing appropriate plasma treatment.

Also, by using the laser light used for checking the plasma application position, costs are greatly reduced. Also, as described above, the laser light is emitted coaxially with the emission axis of the plasma, and the processing target separation distance is calculated based on the laser light emission distance. Therefore, the calculated processing target separation distance matches the plasma emission distance, so the plasma emission distance can be calculated accurately.

Note that, as shown in fig. 5, controller 80 of control device 18 includes calculating section 100 and display control section 102. Calculating section 100 is for calculating the processing target separation distance. Display control section 102 is for causing display lamp 88 to display a light based on the calculated processing target separation distance, and acts as the functional section for reporting the processing target separation distance to the person performing treatment.

Note that, plasma emitting device 10 is an example of a medical-use plasma generating device. Plasma ejecting device 12 is an example of a plasma ejecting device. Laser emitting device 14 is an example of a laser emitting device. Inert gas ejecting device 16 is an example of an inert gas ejecting device. Light receiving element 21 is an example of a light receiving element Cylindrical section 30 is an example of an inner housing. Cooling device 74 is an example of a cooling device. Cylindrical section 76 is an example of an outer housing. Calculating section 100 is an example of a calculating device.

Further, the present disclosure is not limited to the above example embodiments, and various changed or improved methods of embodiment are possible based on the knowledge of someone skilled in the art Specifically, for example, in an embodiment above, whether the calculated processing target separation distance is within a set range is reported to a person performing treatment using display lamp 88, but this may be reported by displaying the value of the calculated processing target separation distance, or by outputting audio of the value of the calculated processing target separation distance.

Also, in an embodiment above, the processing target separation distance is calculated using the phase difference between laser light emitted by laser emitting device 14 and laser light received by light receiving element 21, but the processing target separation distance may be calculated using the time lag between the emission timing of the laser light by laser emitting device 14 and the receiving timing of the laser light by light receiving element 21. Specifically, laser emitting device 14 may emit laser light with a predetermined pulse width and the emission time may be memorized. Also, the receiving time at which light receiving element 21 receives the reflected light may be memorized. Then, the difference between the emission time and the receiving time may be calculated as time lag $\Delta t$, and laser emission distance ($2 \times L_0$) may be calculated using time lag $\Delta t$ according to the formula below.

$$2 \times L_0 = c \times \Delta t$$

wherein c is the speed of light.

Once the laser emission distance ($2 \times L_0$) has been calculated, processing target separation distance $L_1$ can be calculated in a similar manner to that described above.

Further, in an embodiment above, laser light is used as light applied to processing target 68, but various types of light may be used, so long as the light is visible. However, to appropriately check the plasma application position, it is desirable to appropriately calculate the processing target separation distance using reflected light, thus it is desirable to use light with excellent straightness and convergence properties, that is, so-called directional light.

Reference Signs List

10: plasma emitting device (medical-use plasma generating device);
12: plasma ejecting device;
14: laser emitting device (light emitting device);
16: inert gas ejecting device;
21: light receiving element (light receiver);
30: cylindrical section (inner housing);
74: cooling device;
76: cylindrical section (outer housing);
100: calculating section (calculating device)

## Claims

1. A medical-use plasma generating device (10) comprising:

   a plasma ejecting device (12) configured to eject plasma; and
   an inert gas ejecting device (16) configured to eject an inert gas such that an outer circumference of the plasma ejected by the plasma ejecting device (12) is covered by the inert gas, wherein
   the medical-use plasma generating device (10) is provided with

a cylindrical tubular outer housing (76), and
a cylindrical tubular inner housing (30) with an outer dimension smaller than an inner dimension of the outer housing (76), the inner housing (30) being inserted inside the outer housing (76), and

wherein
the plasma ejecting device (12) is configured to eject plasma from the inner housing (30), and the inert gas ejecting device (16) is configured to eject inert gas from between an inner circumferential surface of the outer housing (76) and an outer circumferential surface of the inner housing (30).

**2.** The medical-use plasma generating device according to claim 1, wherein the inert gas ejecting device (16) includes a cooling device (74) configured to cool the inert gas.

**3.** The medical-use plasma generating device according to claim 1 or 2, further comprising:

a light emitting device (14) configured to emit light to a processing target to which plasma is applied, by emitting light in a direction in which the plasma is ejected by the plasma ejecting device (12);
a light receiver (21) configured to receive light emitted from the light emitting device (14) and reflected by the processing target; and
a calculating device (100) configured to calculate a distance between the medical-use plasma generating device (10) and the processing target based on the reflected light received by the light receiver (21).

**4.** The medical-use plasma generating device according to claim 3, wherein
the light receiver (21) is configured to receive light reflected coaxially with respect to an emission axis of the light emitted from the light emitting device (14).

## Patentansprüche

**1.** Vorrichtung (10) zum Erzeugen von Plasma für medizinische Zwecke, die umfasst:

eine Vorrichtung (12) zum Ausstoßen von Plasma, die so ausgeführt ist, dass sie Plasma ausstößt; sowie
eine Vorrichtung (16) zum Ausstoßen von inertem Gas, die so ausgeführt ist, dass sie ein inertes Gas so ausstößt, dass ein Außenumfang des durch die Vorrichtung (12) zum Ausstoßen von Plasma ausgestoßenen Plasmas durch das inerte Gas abgedeckt wird, wobei die Vorrichtung (10) zum Erzeugen von Plasma für medizinische Zwecke versehen ist mit:

einem zylindrischen röhrenförmigen Außengehäuse (76), sowie
einem zylindrischen röhrenförmigen Innengehäuse (30) mit einer Außenabmessung, die kleiner ist als eine Innenabmessung des Außengehäuses (76), wobei das Innengehäuse (30) in das das Innere des Außengehäuses (76) eingeführt ist, und

wobei
die Vorrichtung (12) zum Ausstoßen von Plasma so ausgeführt ist, dass sie Plasma aus dem Innengehäuse (30) ausstößt, und
die Vorrichtung (16) zum Ausstoßen von inertem Gas so ausgeführt ist, dass sie inertes Gas zwischen einer Innenumfangsfläche des Außengehäuses (76) und einer Außenumfangsfläche des Innengehäuses (30) ausstößt.

**2.** Vorrichtung zum Erzeugen von Plasma für medizinische Zwecke nach Anspruch 1, wobei:
die Vorrichtung (16) zum Ausstoßen von inertem Gas eine Kühlvorrichtung (74) enthält, die so ausgeführt ist, dass sie das inerte Gas kühlt.

**3.** Vorrichtung zum Erzeugen von Plasma für medizinische Zwecke nach Anspruch 1 oder 2, des Weiteren umfassend:

eine lichtemittierende Vorrichtung (14), die so ausgeführt ist, dass sie Licht zu einem Bearbeitungs-Objekt emittiert, auf das Plasma aufgebracht wird, indem sie Licht in einer Richtung emittiert, in der das Plasma durch die Vorrichtung (12) zum Ausstoßen von Plasma ausgestoßen wird;
eine Lichtempfangseinrichtung (21), die so ausgeführt ist, dass sie Licht empfängt, das von der lichtemittierenden Vorrichtung (14) emittiert und von dem Bearbeitungs-Objekt reflektiert wird; sowie
eine Berechnungsvorrichtung (100), die so ausgeführt ist, dass sie einen Abstand zwischen der Vorrichtung (10) zum Erzeugen von Plasma für medizinische Zwecke und dem Bearbeitungs-Objekt auf Basis des durch die Lichtempfangseinrichtung (21) empfangenen reflektierten Lichts berechnet.

**4.** Vorrichtung zum Erzeugen von Plasma für medizinische Zwecke nach Anspruch 3, wobei die Lichtempfangseinrichtung (21) so ausgeführt ist, dass sie Licht empfängt, das in Bezug auf eine Emissionsachse des von der lichtemittierenden Vorrichtung (14) emittierten Lichts koaxial reflektiert wird.

## Revendications

1. Dispositif de génération de plasma d'usage médical (10) comprenant :

   un dispositif d'éjection de plasma (12) configuré pour éjecter un plasma, et
   un dispositif d'éjection de gaz inerte (16) configuré pour éjecter un gaz inerte de sorte à ce que la circonférence externe du plasma éjecté par le dispositif d'éjection de plasma (12) soit recouverte par le gaz inerte, où
   le dispositif de génération de plasma d'usage médical (10) est muni :

   d'une enveloppe externe tubulaire cylindrique (76), et
   d'une enveloppe interne tubulaire cylindrique (30) dont la dimension externe est plus petite que la dimension interne de l'enveloppe externe (76), l'enveloppe interne (30) étant insérée à l'intérieur de l'enveloppe externe (76), et où
   le dispositif d'éjection de plasma (12) est configuré pour éjecter le plasma de l'enveloppe interne (30), et
   le dispositif d'éjection de gaz inerte (16) est configuré pour éjecter le gaz inerte depuis la partie située entre la surface circonférentielle interne de l'enveloppe externe (76) et la surface circonférentielle externe de l'enveloppe interne (30).

2. Dispositif de génération de plasma d'usage médical (10) selon la revendication 1, dans lequel :
   le dispositif d'éjection de gaz inerte (16) inclut un dispositif de refroidissement (74) configuré pour refroidir le gaz inerte.

3. Dispositif de génération de plasma d'usage médical (10) selon la revendication 1 ou la revendication 2, comprenant en outre :

   un dispositif d'émission de lumière (14) configuré pour émettre une lumière vers une cible de traitement à laquelle est appliquée le plasma, en émettant la lumière dans la direction dans laquelle le plasma est éjecté par le dispositif d'éjection de plasma (12),
   un récepteur de lumière (21) configuré pour recevoir la lumière émise depuis le dispositif d'émission de lumière (14) et réfléchie par la cible de traitement, et
   un dispositif de calcul (100) configuré pour calculer la distance entre le dispositif de génération de plasma d'usage médical (10) et la cible de traitement sur la base de la lumière réfléchie reçue par le récepteur de lumière (21) .

4. Dispositif de génération de plasma d'usage médical (10) selon la revendication 3, dans lequel :
   le récepteur de lumière (21) est configuré pour recevoir la lumière réfléchie coaxiale à l'axe d'émission de la lumière émise par le dispositif d'émission de lumière (14).

FIG. 1

28
14
72
32
34
21
74
24
52
20
52
78
26
52
16
50
70
56
50
10
56
50
12
56
22
30
60
76

FIG. 2

14
67
32
34
21
62
52
24
20
52
12
56
52
58
56
58
56
50
58
50
22
50
60
30

FIG. 3

14
34
69
21
32
78
70
$L_0$
$L_2$
20
76
30
$L_1$
68

FIG. 4
78
76
70
16

FIG. 5

Plasma emitting device
10
Electrode
22,24
Processing gas supply device
28
Laser emitting device
14
Inert gas supply device
72
Cooling device
74
Light receiving element
21
Display lamp
88
Control device
18
Drive circuit
82
Control circuit
86
Controller
80
Calculating section
100
Display control section
102

FIG. 6
20(12)
30
70(16)
76

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20130108797 A
- JP 2012059548 A
- JP 2010129198 A